# EUROPEAN PATENT SPECIFICATION

(11) **EP 2 791 839 B1**
(45) Date of publication and mention of the grant of the patent: **25.05.2022**
(21) Application number: 12857166.8
(22) Date of filing: 17.12.2012
(51) Int. Cl.: G16B 20/10

(54) **MATHEMATICAL NORMALIZATION OF SEQUENCE DATA SETS**
MATHEMATISCHE NORMALISIERUNG VON SEQUENZDATENSÄTZEN
NORMALISATION MATHÉMATIQUE D'ENSEMBLES DE DONNÉES DE SÉQUENCE

(30) Priority: 17.12.2011 US 201161577013 P
(43) Date of publication of application: 22.10.2014
(73) Proprietor: Roche Diagnostics GmbH, 68305 Mannheim (DE); F. Hoffmann-La Roche AG, 4070 Basel (CH)
(72) Inventor: OLIPHANT, Arnold, San Jose, CA 95138 (US); SPARKS, Andrew, San Jose, CA 95138 (US); WANG, Eric, San Jose, CA 95138 (US); STRUBLE, Craig, San Jose, CA 95138 (US)
(74) Representative: Mewburn Ellis LLP
(86) International application number: PCT/US2012/070177
(87) International publication number: WO 2013/090925

(56) References cited:
- WO-A1-2011/060240
- US-A1- 2003 003 459
- ERIC Z. CHEN ET AL: "Noninvasive Prenatal Diagnosis of Fetal Trisomy 18 and Trisomy 13 by Maternal Plasma DNA Sequencing", PLOS ONE, vol. 6, no. 7, 6 July 2011 (2011-07-06), page e21791, XP055024137, DOI: 10.1371/journal.pone.0021791
- BOLSTAD B M ET AL: "A COMPARISON OF NORMALIZATION METHODS FOR HIGH DENSITY OLIGONUCLEOTIDE ARRAY DATA BASED ON VARIANCE AND BIAS", BIOINFORMATICS, OXFORD UNIVERSITY PRESS, SURREY, GB, vol. 19, no. 2, 22 January 2003 (2003-01-22), pages 185-193, XP008041261, ISSN: 1367-4803, DOI: 10.1093/BIOINFORMATICS/19.2.185
- HAHN S ET AL: "Determination of fetal chromosome aberrations from fetal DNA in maternal blood: has the challenge finally been met?", EXPERT REVIEWS IN MOLECULAR MEDICINE, CAMBRIDGE UNIVERSITY PRESS, CAMBRIDGE, vol. 13, 4 May 2011 (2011-05-04), pages e16-1, XP002685359, ISSN: 1462-3994, DOI: 10.1017/S1462399411001852
- GARY J W LIAO ET AL: "Targeted massively parallel sequencing of maternal plasma DNA permits efficient and unbiased detection of fetal alleles", CLINICAL CHEMISTRY, P.B. HOEBER , vol. 57, no. 1 1 January 2011 (2011-01-01), pages 92-101, XP002674771, ISSN: 0009-9147, DOI: 10.1373/CLINCHEM.2010.154336 Retrieved from the Internet: URL:http://www.clinchem.org/content/57/1/9 2 [retrieved on 2010-11-15]
- Kemperman J. H. B.: "Least absolute value and median polish" In: "Inequalities in Statistics and Probability : Proceedings of the Symposium on Inequalities in Statistics and Probability, October 27-30, 1982, Lincoln, Nebraska", 1 January 1984 (1984-01-01), Institute of Mathematical Statistics, Hayward, CA, XP055779023, ISSN: 0749-2170 ISBN: 978-0-940600-04-1 vol. 5, pages 84-103, DOI: 10.1214/lnms/1215465633,

## Description

### CROSS-REFERENCE TO RELATED APPLICATIONS

### FIELD OF THE INVENTION

This invention relates to methods for optimizing data in multiplexed data sets.

### BACKGROUND OF THE INVENTION

In the following discussion certain articles and methods will be described for background and introductory purposes. Nothing contained herein is to be construed as an "admission" of prior art. Applicant expressly reserves the right to demonstrate, where appropriate, that the articles and methods referenced herein do not constitute prior art under the applicable statutory provisions.

Detection of nucleic acid levels in biological samples has wide applicability in numerous areas of biological enquiry. Identification of nucleic acid levels in a sample, including levels of DNA associated with copy number variation and levels of RNA associated with gene expression, can be used in diagnostic and prognostic methods. Variability in detection levels of biological molecules introduced by experimental conditions decreases the quality of analysis, and thus it is desirable to reduce such variability.

Generally, variability in detection of biological molecules can be decreased using external reference sets that have known levels of nucleic acids and use substantially similar reagents and conditions. However, if a reference demonstrates substantial batch effects or lab to lab systemic effects, this may unnecessarily inflate apparent variability and lead to erroneous results.

There is thus a need for improved processes for decreasing variability in data sets for detection of nucleic acid levels in a biological molecule.

Chen et al. PLoS ONE 6(7): e21791 (2011) describes noninvasive prenatal diagnosis of fetal trisomy 18 and trisomy 13 by maternal plasma DNA sequencing. In particular, the study employed massively parallel sequencing of DNA molecules in the plasma a pregnant women, followed by sequencing data analysis and detection of trisomy 13 and trisomy 18 samples. Improvements in diagnostic performance were reported as a result of using a non-repeat-masked reference genome and as a result of using a GC-corrected read count. The GC correction step aimed to eliminate the effect of the GC bias on the sequenced read counts. For each sample, all chromosomes were first bioinformatically divided in segments of the same size (50 kb bins). A LOESS regression was applied to fit the number of sequenced reads in each bin against the GC content of the corresponding bin. Chen et al., does not disclose a method which involves normalizing the frequency of a sequence of individual loci within the dataset and subjecting the detected frequencies of a sequence from an individual locus across all samples to a mathematical transformation based on the frequencies of sequence of the at least 15 other loci across all samples within the data set.

WO2011/060240 A1 describes a method of analyzing copy number of a target locus, e.g. by RT-PCR. In some embodiments, the method involves determining the difference (delta Ct) between cycle number of a target locus and the cycle number of one or more reference loci. In some embodiments, the method involves normalizing the difference in cycle numbers using a calibrator based on the 80% trimmed means of the delta Ct for a multi-well plate. WO2011/060240 A1 does not disclose a method for the identification of one or more chromosomal abnormalities in a fetus in which a data set comprising frequency sequence data for a set of at least 16 loci from a single chromosome from two or more maternal samples each comprising both fetal and maternal nucleic acids are determined in a single sequencing reaction. Moreover, the method of WO2011/060240 A1 does not involve subjecting the detected frequencies of a sequence from an individual locus across all samples to a mathematical transformation based on the frequencies of sequences of the at least 15 other loci across all samples within the data set using a regression technique to normalize the frequency of the set of loci. WO2011/060240 A1 does not disclose identifying differences in sequence frequencies of the normalized loci from the fetal chromosome relative sequence frequencies of loci of one or more other chromosomes in one or more of said maternal samples.

### SUMMARY OF THE INVENTION

The present invention provides a computer implemented process for the identification of the presence or absence of one or more chromosomal abnormalities in a fetus using a maternal sample comprising both fetal and maternal nucleic acids, the process comprising:
i) providing a data set comprising frequency sequence data for a set of at least 16 loci from a single chromosome from two or more maternal samples each comprising both fetal and maternal nucleic acids, wherein each sample is identified by a sample index, and wherein the sequences of the dataset are determined in a single sequencing reaction;
ii) normalizing the frequency of a sequence of individual loci within said dataset, wherein locus or loci refers to a nucleic acid region of known location in a genome, and wherein the normalizing comprises:
   subjecting the detected frequencies of a sequence from an individual locus across all samples to a mathematical transformation based on the frequencies of sequences of the at least 15 other loci across all samples within the data set, to reduce experimentally introduced variation, wherein the mathematical transformation comprises using a regression technique to normalize the frequency of the set of loci; and
iii) identifying differences in sequence frequencies of a normalized locus from the fetal chromosome relative to sequence frequencies of loci of one or more other chromosomes in one or more of said maternal samples, thereby identifying the presence or absence of one or more chromosomal abnormalities in a fetus.

In some embodiments, the nucleic acids are DNA. In some embodiments, the regression technique is multiple linear regression or median polish.

In some embodiments, the sequences of the individual loci within the data set have been subjected to an amplification reaction prior to sequence determination.

In some embodiments, the amplification reaction uses primers that comprise both an index region and a sequence region that specifically hybridizes to the individual locus.

In some embodiments, the sequence data on at least 64 loci, at least 384 loci or at least 768 loci are used in the process.

The application describes a system and processes for normalizing frequency data within a single data set using data generated from multiplexed sequencing systems. More particularly, the present application describes processes to identify differences in sequence frequencies of a locus, a sample, and/or a grouping of multiple loci (*e.g*., a chromosome or sub-chromosomal region) relative to the other sequences in a multiplexed set of sequence data. The processes utilize this information to minimize empirically-introduced differences present in sequences within the multiplexed set of sequence data. The multiplexed systems provide an integrated means for distinguishing between the individual molecules being sequenced, e.g., through the use of indices associated with a locus, sample and/or chromosome and/or sequence differences inherent in different genomic regions, allowing for the simultaneous processing of sequences under the same conditions.

The three primary dimensions of the genetic data sets that can be normalized using the processes describes herein are locus normalization, sample normalization and chromosome normalization. Locus normalization and sample normalization generally utilize 16 or more sequences to normalize any given dimension of the data set, although additional sequences for use in the processes are generally preferable. For instance, although 16 sequences can be used for the normalization process, it is preferable to use at least 32, more preferably 64, more preferably 128, more preferably 256, more preferably 384, more preferably 512, more preferably 640, even more preferably 768 loci determined.

Where two dimensions are being simultaneously normalized, such as normalization of locus effects and sample effects in a data set, the number of sequences that need to be normalized would be at least 16 (for the first dimension) ^{∗} 16 (for the second dimension), so the normalization process would require at least 256 sequences to normalize, e.g., sequences from a single chromosome. For normalization at the chromosome level, the number of sequences used in the normalization process would be 16^{∗}(16^{∗}n) = 256^{∗}n sequences, where n is the number of chromosomes interrogated.

The sequences of the multiplexed data set are determined in a single sequencing reaction, e.g., using a single slide or flow cell. More preferably, the levels of the individual sequences of the data set are determined using a single physical feature (*e.g*., lane, channel or nanopore) via use of next generation sequencing technology.

Described herein is a computer implemented process for the normalization of the frequency data of an individual sequence within a single multiplexed data set, comprising: providing a multiplexed data set having frequency data on at least 16 biological molecules, and subjecting the detected frequency of a sequence from an individual biological molecule to a mathematical transformation based on the frequency of at least 15 other sequences within the data set to reduce experimentally introduced variation of the sequence. This variation can be based on an assumption of expected behavior for those particular sequences. In certain aspects, data on sequences with behavior well outside the expected are masked during the normalization process to improve results. For example, if a locus is more or less efficient than other loci that predicted to have the same behavior, the frequency for that locus can be normalized to be more like the other loci. Similarly, for samples, the frequency of sequences detected per locus within a genetic class (e.g. chromosome or genomic region in the same sample) can be made equivalent, because samples should have the same "typical" frequency per locus in order to make a meaningful comparison.

In certain aspects, the application describes processes for normalization of sequence levels in a multiplexed data set on a locus basis. Such normalization provides adjustment of sequence frequency of individual loci within a data set via comparison to sequence frequency of other individual loci within the set. Certain loci will display effects due to differences of assay efficiencies, including hybridization, ligation, and extension under certain experimental condition as compared to other loci. These loci are measured across all of the sequences in a single reaction, preferably a single sequencing lane, to obtain the frequency of each loci within the reaction, and require a minimum number of unique loci or samples (e.g., 16 or more) to measure accurately. The average frequency of each locus can be calculated e.g., as mean frequencies or using robust statistical techniques such as trimmed means. Preferably, the levels are estimated using techniques such as regression techniques, e.g., multiple linear regression or median polish.

The application also describes normalization of sequence levels in a multiplexed data set on a sample basis. Such normalization provides adjustment of sequence levels of loci from a particular sample within a data set via comparison to sequence levels of other individual loci from one or more other samples within the multiplexed data set. Preferably, levels from at least 16 different loci present in the individual samples are used for the sample normalization processes.

Thus, in a specific example, the application describes a computer implemented process for the normalization of the levels of an individual sequence within a multiplexed data set, comprising providing a multiplexed data set having sequence data on a set of at least 16 loci from one sample or one locus from 16 samples, determining the frequency or number of counts of each locus within that data set, calculating the average frequency data, comparing the average frequency data on the set of loci to the frequency of an individual locus to identify differences in the frequency of the individual locus compared to the set of loci, and adjusting the frequency of the individual sequence based on the frequency data of the set of loci to reduce experimentally introduced variation. These processes are useful in normalizing individual loci either within a single sample or the same locus present in multiple samples.

With respect to the loci interrogated in the processes the individual loci may be loci with different and distinguishable sequences from a single or small number of samples, e.g., 16 or more loci with different sequences from a single sample. Alternatively, the different loci used in the processes may include loci with the same or indistinguishable sequences, but which are distinguishable in the process based on their source, *e.g.,* the same locus is used from different samples, and they are associated with indices that allow differentiation of the same locus from the different samples.

In a specific aspect, the same locus from multiple samples can be normalized using the methods More specifically, the application describes a computer-implemented process for the normalization of frequency data from sequences of two or more loci within a single multiplexed data set, comprising providing a multiplexed data set comprising sequence data on two or more individual loci from at least 16 different samples, summing the sequence data from the loci of the multiple samples to identify overall frequency differences of the loci from the different samples, and subjecting the summed data to a mathematical transformation to correct the overall frequency of the loci from one sample based on a comparison to the summed biological levels of the loci in the other samples.

The levels of nucleic acids within the multiplexed data set can be used to determine a mean or a median that provides an established reference point. Thus, in some aspects the sequencing counts per sample are standardized so that the median per locus sequencing counts are scaled to such an established reference point. This allows samples to be compared to one another to determine more physiologically meaningful data.

In other aspects, the processes described herein utilize normalization on a per sample basis to address issues with differences in conditions, including conditions that may have arisen from the samples being prepared separately before pooling into a single sequencing reaction. More specifically, the application describes a computer-implemented process for the normalization of frequency data from loci of two or more samples within a single multiplexed data set, comprising providing a multiplexed data set comprising sequence data on 16 or more individual loci from at least two samples, summing the sequence data from the loci to identify overall frequency differences of the loci from the different samples, and subjecting the summed data to a mathematical transformation to correct the overall frequency of the loci from one sample based on a comparison to the summed biological levels of the loci in the other samples.

In other aspects, the processes are used to remove chromosome effects. Median chromosome counts for each chromosome across all samples in the lane are set to a reference value. Total counts per chromosome are preferably preserved so only the variance is reduced. Such normalization provides adjustment of sequence levels of loci from a single chromosome within a data set via comparison to sequence levels of other individual chromosomes within the multiplexed data set. Preferably, frequency data from at least 16 different loci present in the individual samples are used for the chromosome normalization processes.

In certain preferred aspects, the application describes for normalization of sequence frequency data in a multiplexed data set on a dual basis, *e.g.,* a locus by sample basis or a locus by chromosome basis. This allows certain data, including e.g., sample by chromosome interactions and per sample residuals, to be maintained; these biological effects and variances can be used to determine copy number of larger regions and to measure surety.

The processes of the invention are used to normalize sequencing data to determine a fetal chromosomal abnormality (e.g., a trisomy or monosomy) in a mixed sample. In this aspect, the present invention provides processes to identify differences in sequence frequencies of loci from a fetal chromosome region relative to one or more other chromosomes in one or more maternal samples using a multiplexed set of sequence data. The processes of the invention utilize this information to minimize empirically-introduced differences present in sequences from these genomic regions, and optimize the identification of potential duplications, deletions, and/or aneuploidies within the multiplexed set of sequence data. The multiplexed systems of the invention provide an integrated means for distinguishing between the individual molecules being sequenced from different samples, e.g., through the use of indices associated with a sample, allowing for the simultaneous interrogation of chromosomal abnormalities in two or more samples under the same conditions.

Such normalization of samples and/or chromosomes can be performed using various characteristics of the sequences in addition to the sequence frequency data. In one example, known biological activity associated with different sequences in the multiplexed data set can be used in determining the sequences of the data set used in the normalization process for samples. In yet another example, positional effects of certain loci within the chromosomal context can be used in determining the sequences of the data set used in the normalization process for samples. In still other examples, normalization can be based on the selection of certain classes of loci, *e.g*., housekeeping genes or other loci that are known to display minimal variability between samples.

In certain aspects, the individual sequences within a multiplexed data set may be subjected to an amplification reaction of the individual molecules prior to sequence determination. The application thus describes processes for quantifying nucleic acid sequences present in a single, multiplexed data set that have been subjected to such amplification. Specifically, the application describes systems and processes comprising the steps of: amplifying at least 16 biological molecules; sequencing the amplification products of the at least 16 biological molecules in a single, multiplexed data set, wherein the sequencing data is indicative of a detected quantity of progeny sequences arising from amplification of the individual sequences in the set; comparing the sequence data frequency on the biological molecules to the frequency of an individual sequence to identify overall differences in the sequence levels of the biological molecules, and subjecting the detected frequency of the individual sequences to a mathematical transformation based on the frequency data of at least 16 other sequences within the data set to reduce experimentally introduced variation in the frequency data of the biological molecules.

In some aspects, processes use internal comparators within each data set to provide normalization quotients, and the detected levels of each biological molecule within the data set can be corrected based on normalization quotient for data in the data set corresponding to that particular sample.

In another aspect, the application describes a process for quantifying at least one nucleic acid of unknown concentration in a multiplexed sequencing data set. Thus the application provides a computer implemented process for quantifying at least one nucleic acid of unknown concentration in a multiplexed sequencing data set, comprising providing a multiplexed data set having sequence data on at least 16 biological molecules, comparing the frequency data on the biological molecules to the frequency of an individual sequence to identify overall differences in the frequencies of the biological molecules, subjecting the detected level of the individual sequence to a mathematical transformation based on the level of at least 15 other sequences within the data set to reduce experimentally introduced variation, and quantifying the nucleic acid of unknown concentration based on the transformed level.

In some aspects, the processes described herein can be used to normalize data values between samples. These processes utilize 16 or more different values, preferably 32 or more different values, more preferably 48 or more different values, more preferably 64 or more different values, and most preferably 96 or more different values that correspond to frequency of individual nucleic acids from two or more biological molecules within the data set. The processes use internal comparators within each data set to provide normalization quotients, and the detected frequency data of each nucleic acid within the data set can be corrected based on normalization quotient for that particular data set.

In other aspects, the processes can be used to normalize data values between nucleic acids from a single sample. These processes utilize 16 or more different values, preferably 32 or more different values, more preferably 48 or more different values, more preferably 64 or more different values, more preferably 96 or more different values, more preferably 128 or more different values, more preferably 256 or more different values, more preferably 384 or more different values, more preferably 512 or more different values, more preferably 640 or more different values, even more preferably 768 or more different values that correspond to frequency data of individual nucleic acids from a single sample in the data set.

These aspects and other features and advantages of the invention are described in more detail below.

### DEFINITIONS

The terms used herein are intended to have the plain and ordinary meaning as understood by those of ordinary skill in the art. The following definitions are intended to aid the reader in understanding the present invention, but are not intended to vary or otherwise limit the meaning of such terms unless specifically indicated.

The term "amplified" as used herein refers to the any biological molecule whose amount has been increased at least two fold by any nucleic acid amplification or replication process performed *in vitro* as compared to its starting amount.

As used herein "amplification" refers to a technique for replicating a specific piece of target DNA *in vitro,* even in the presence of excess non-specific DNA. One example of amplification is through the use of the polymerase chain reaction, or "PCR". Primers are added to the target DNA, where the primers initiate the copying of the target DNA using nucleotides and, typically, Taq polymerase or the like. By cycling the temperature, the target DNA is repetitively denatured and copied. A single copy of the target DNA, even if mixed in with other, random DNA, can be amplified to obtain replicates. The polymerase chain reaction can be used to detect and measure very small amounts of DNA and to create customized pieces of DNA. In some instances, linear amplification processes may be used as an alternative to PCR.

The term "biological molecule" refers to any sample comprising all or a portion of the genetic information of an organism, including but not limited to virus, bacteria, fungus, plants and animals, and in particular mammals. The genetic information that can be interrogated using the processes of the invention includes genomic DNA (both coding and non-coding regions), mitochondrial DNA, RNA, and nucleic acid products derived from each of these. Such nucleic acid products include cDNA created from mRNA or products of pre-amplification to increase the material for analysis.

The term "chromosomal abnormality" refers to any genetic variation that affects all or part of a chromosome larger than a single locus. The genetic variants may include but not be limited to any copy number variant such as duplications or deletions, translocations, inversions, and mutations. Examples of chromosomal abnormalities include, but are not limited to, Down Syndrome (Trisomy 21), Edwards Syndrome (Trisomy 18), Patau Syndrome (Trisomy 13), Klinefelter's Syndrome (XXY), Triple X syndrome, XYY syndrome, Trisomy 8, Trisomy 16, Turner Syndrome (XO), Robertsonian translocation, DiGeorge Syndrome and Wolf-Hirschhorn Syndrome.

The term "identification index" refers generally to a series of nucleotides that are incorporated into an oligonucleotide during oligonucleotide synthesis for identification purposes. Identification index sequences are preferably 6 or more nucleotides in length. In a preferred aspect, the identification index is long enough to have statistical probability of labeling each molecule with a target sequence uniquely. For example, if there are 3000 copies of a particular target sequence, there are substantially more than 3000 identification indexes such that each copy of a particular target sequence is likely to be labeled with a unique identification index. The identification index may contain additional nucleotides that allow for identification and correction of sequencing errors including the detection of deletion, substitution, or insertion of one or more bases during sequencing as well as nucleotide changes that may occur outside of sequencing such as oligo synthesis, amplification, and any other aspect of the assay. The index may be combined with any other index to create one index that provides information for two properties (e.g., sample-identification index, allele-locus index).

The term "expected behavior" as used herein in refers to the predicted behavior of a particular sequence or set of sequences based on biological differences or similarities. For example, an expectation can be made that the capture and sequencing of the same locus from different samples should result in the same frequency of sequences. In another example, different molecules from the same sample should have predicted relative amounts.

The terms "locus" and "loci" as used herein refer to a nucleic acid region of known location in a genome.

The term "locus index" refers generally to a series of nucleotides that correspond to a given genomic locus. In a preferred aspect, the locus index is long enough to label each target sequence region uniquely. For instance, if the method uses 192 target sequence regions, there are at least 192 unique locus indexes, each uniquely identifying each target region. The locus index may contain additional nucleotides that allow for identification and correction of sequencing errors including the detection of deletion, substitution, or insertion of one or more bases during sequencing as well as nucleotide changes that may occur outside of sequencing such as oligo synthesis, amplification, and any other aspect of the assay. The index may be combined with any other index to create one index that provides information for two properties (e.g. sample-identification index, allele-locus index).

The term "maternal sample" as used herein refers to any sample taken from a pregnant mammal which comprises both fetal and maternal genomic material (e.g., DNA or RNA). Maternal samples may comprise cells from both the mother and fetus, or alternatively cell free DNA or RNA from the mother and the fetus. Preferably, maternal samples for use in the invention are obtained through relatively non-invasive means, e.g., phlebotomy or other standard techniques for extracting peripheral samples from a subject.

The term "multiplexed data set" refers to any data set from different samples, loci, and/or chromosomes in which 16 or more elements of the data set are analyzed simultaneously where the processing or detection uses a biochemical process (*e.g*., amplification), all or part of the processing or detection may take place in a single vessel, e.g., amplification of the different samples within a well of multiwall plate, within a single tube, on a single slide, in a single flowcell, or the like. In other aspects, a multiplexed data set may be detected using a single physical lane or channel in a sequencing reaction, such as detection of multiple samples on a specific sequencing slide or flow cell. In more specific aspects, multiplexed data set may be detected using detection of multiple samples within one data generation set (e.g., within a single lane) on a sequencing slide.

The terms "sequencing" as used herein refers generally to any and all biochemical methods that may be used to determine the order of nucleotide bases including but not limited to adenine, guanine, cytosine and thymine, in one or more molecules of DNA. As used herein the term "sequence determination" means using any method of sequencing known in the art to determine the sequence nucleotide bases in a nucleic acid.

The term "sample index" refers generally to a series of unique nucleotides (i.e., each sample index is unique), and can be used to allow for multiplexing of samples such that each sample can be identified based on its sample index. In a preferred aspect, there is a unique sample index for each sample in a set of samples, and the samples are pooled during sequencing. For example, if twelve samples are pooled into a single sequencing reaction, there are at least twelve unique sample indexes such that each sample is labeled uniquely. The sample index may contain additional nucleotides that allow for identification and correction of sequencing errors including the detection of deletion, substitution, or insertion of one or more bases during sequencing as well as nucleotide changes that may occur outside of sequencing such as oligo synthesis, amplification, and any other aspect of the assay. The index may be combined with any other index to create one index that provides information for two properties (e.g., sample-identification index, allele-locus index).

### BRIEF DESCRIPTION OF THE FIGURES

FIG. 1 is a block diagram illustrating an exemplary system environment.
FIGs. 2A and 2B are box-plots illustrating removal of sequence bias caused by sample effects.
FIGs. 3A and 3B is a graph illustrating removal of bias caused by locus effects.
FIGs. 4A and 4B is a graph illustrating chromosome level normalization.
FIGs. 5A and 5B is a graph illustrating a first example of normalization of sequence data based on chromosome position effects.
FIGs. 6A and 6B is a graph illustrating a second example of normalization of sequence data based on chromosome position effects.

### DETAILED DESCRIPTION OF THE INVENTION

The practice of the techniques described herein may employ, unless otherwise indicated, conventional techniques and descriptions of organic chemistry, polymer technology, molecular biology (including recombinant techniques), cell biology, biochemistry, and sequencing technology, which are within the skill of those who practice in the art. Such conventional techniques include polymer array synthesis, hybridization and ligation of polynucleotides, and detection of hybridization using a label. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual; Dieffenbach, Dveksler, Eds. (2003), PCR Primer: A Laboratory Manual; Bowtell and Sambrook (2003), DNA Microarrays: A Molecular Cloning Manual; Mount (2004), Bioinformatics: Sequence and Genome Analysis; Sambrook and Russell (2006), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W.H. Freeman, New York N.Y.; Gait, "Oligonucleotide Synthesis: A Practical Approach" 1984, IRL Press, London; Nelson and Cox (2000), Lehninger, Principles of Biochemistry 3rd Ed., W. H. Freeman Pub., New York, N.Y.; and Berg et al. (2002) Biochemistry, 5th Ed., W.H. Freeman Pub., New York, N.Y.,

Note that as used herein and in the appended claims, the singular forms "a," "an," and "the" include plural referents unless the context clearly dictates otherwise. Thus, for example, reference to "a sequence" refers to one or more copies of a sequence, and reference to "the process" includes reference to equivalent steps and methods known to those skilled in the art, and so forth.

Unless defined otherwise, all technical and scientific terms used herein have the same meaning as commonly understood by one of ordinary skill in the art to which this invention belongs.

Where a range of values is provided, it is understood that each intervening value, between the upper and lower limit of that range and any other stated or intervening value in that stated range is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either both of those included limits are also included in the invention.

In the following description, numerous specific details are set forth to provide a more thorough understanding of the present invention. However, it will be apparent to one of skill in the art that the present invention may be practiced without one or more of these specific details, unless required by the claims. In other instances, well-known features and procedures well known to those skilled in the art have not been described in order to avoid obscuring the invention.

### The Invention in General

The present invention relates to methods of providing quality control metrics to multiplexed sequencing data sets. More specifically, the processes of the present invention relate to normalization procedures for sequences within multiplexed data sets using the sequence information from multiplexed sequencing data set itself rather than the utilization of any external references. Such metrics are useful for correcting variations in the individual sequencing frequency data that are introduced by variation in experimental conditions rather than variation that reflects biological differences. This provides for more accurate data, *e.g.,* for use in analysis of comparative nucleic acid sequencing frequency data for RNA expression or genetic copy number analysis.

In certain aspects, the sequence data of the multiplexed data set is used to determine biologically relevant increases or decreases in representation for a given locus, sample or chromosome. If the sequence counts of certain loci or samples are increasing or decreasing for a reason other than the relevant biological process under investigation, including, e.g., variation due to experimental conditions or other unrelated biological effects, the processes of the invention can be used to remove these artifacts without perturbing the underlying relevant biological variation.

The processes of the present invention are in contrast to more conventional methods of measuring variability relative to an external reference set that has analyzed similar samples or loci under roughly the same experimental conditions. The ability of the processes of the invention to do "real time" normalization for one or more sequences in a data set by comparison with the other sequence data within the set that were identified under identical conditions reduces variability that may be introduced by even relatively minor changes in sample preparation and/or processing. The measurements of different sequences isolated and/or amplified from a nucleic acid (*e.g*., using PCR can be enhanced by reducing or removing the technical variability using such processes. Thus, the present invention provides methods to mitigate effects that are not due purely to the biology of the sample and/or genomic region under investigation.

In the multiplexed data sets of the invention, samples are preferably processed under the same conditions, and sequences are determined in a single sequencing reaction. In preferred aspects, different samples may be processed during the initial part of the assay as separate samples. Those samples may be labeled with identifying sample indices and then pooled together before the sequence detection step.

The processes of the invention utilize observed variances within and between multiplexed samples in a single sequencing determination reaction, e.g., individual sequences are run within a single sequencing lane and differentiated using an identifier such as an index. This is in contrast to more conventional methods of normalization, which require estimating variance based upon information obtained from a previously analyzed reference dataset. The processes of the invention can thus leverage the observed variances within and between samples in a single sequencing lane rather than estimating variance based upon information obtained from a previously analyzed reference dataset. By utilizing information from the multiplexed data set, the processes of the invention are less susceptible to process drift, and do not require external reference samples or normalizing adjustments based upon historical information.

In certain aspects, biases caused by chromosomal position can be removed from the individual multiplexed data sets using a robust regression technique. An example of such a technique is a "smoothing" of the sequencing counts obtained for each sample sequenced to remove positional biases, *e.g.,* using a robust regression technique (*e.g*., LOWESS piecewise linear regression). When using such a technique, preferably total counts per chromosome are preserved so that only variance is reduced between samples in a data set. Additional normalizations that might be incorporated include adjusted polymorphic counts due to measured allelic specificity estimated by oligonucleotide variation and the presence or absence of polymorphisms.

### Computer implementation of the Processes of the Invention

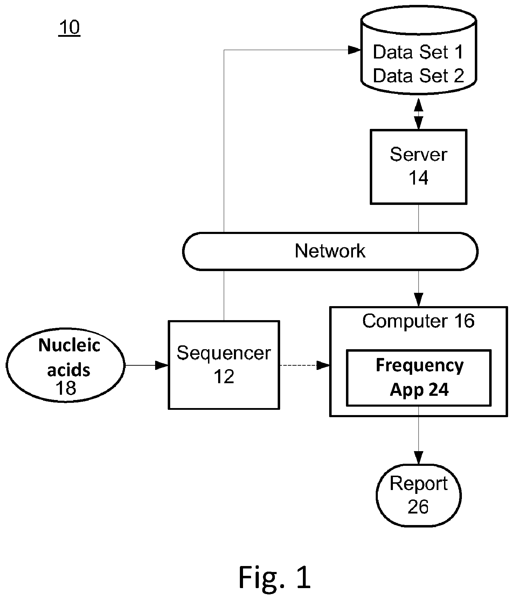

FIG. 1 is a block diagram illustrating an exemplary system environment in which the processed of the present invention may be implemented for normalizing levels between loci, samples, and/or chromosomes. The system 10 includes a server 14 and a computer 16, and preferably these are associated with a DNA sequencer 12. The DNA sequencer 12 may be coupled to the server 14 and/or the computer directly or through a network. The computer 16 may be in communication with the server 14 through the same or different network.

In one embodiment, a set of 16 or more nucleic acids or progeny thereof 18 are input to the DNA sequencer 12. The DNA sequencer 12 may be any commercially available instrument that automates the DNA sequencing process for sequence analysis of nucleic acids representative of a biological molecule 18. The output of the DNA sequencer 12 may be in the form of a multiplexed data sets 20 comprising frequency data for loci and/or samples that are distinguishable based on associated indices. In one embodiment, the multiplexed data set 20 may be stored in a database 22 that is accessible by the server 14.

According to the exemplary embodiment, the computer 16 executes a software component 24 that calculates frequency data for the different nucleic acids in the multiplexed data set 18. In one embodiment, the computer 16 may comprise a personal computer, but the computer 16 may comprise any type of machine that includes at least one processor and memory.

The output of the software component 24 comprises a report 26 with adjusted frequency data for one or more sequences within the multiplexed data set. The report 26 may be paper that is printed out, or electronic, which may be displayed on a monitor and/or communicated electronically to users via e-mail, FTP, text messaging, posted on a server, and the like.

Although the normalization process of the invention is shown as being implemented as software 24, it can also be implemented as a combination of hardware and software. In addition, the software 24 for normalization may be implemented as multiple components operating on the same or different computers.

Both the server 14 and the computer 16 may include hardware components of typical computing devices (not shown), including a processor, input devices (e.g., keyboard, pointing device, microphone for voice commands, buttons, touchscreen, etc.), and output devices (e.g., a display device, speakers, and the like). The server 14 and computer 16 may include computer-readable media, e.g., memory and storage devices (e.g., flash memory, hard drive, optical disk drive, magnetic disk drive, and the like) containing computer instructions that implement the functionality disclosed when executed by the processor. The server 14 and the computer 16 may further include wired or wireless network communication interfaces for communication.

### Use of Indices in the Assay Systems of the Invention

The sequences in the multiplexed data set are associated with one or more indices that are identifying for a particular sample being analyzed and optionally for a locus. These indices are preferably associated with the selected nucleic acids during an amplification step using primers that comprise both the index and sequence regions that specifically hybridize to the nucleic acid region. Such indices allow the differentiation of individual sequences within the multiplexed data set (or progeny thereof), and allow for more efficient use of the sequencing technology.

In one example, the primers used for amplification of biological molecules are designed to provide a locus index between the selected nucleic acid region primer region and a universal amplification region. The locus index is unique for each selected nucleic acid region and representative of a locus on a chromosome of interest or reference chromosome, so that quantification of the locus index in a sample provides quantification data for the locus and the particular chromosome containing the locus. The universal amplification region can be used to provide additional amplification of the individual sequences in a single amplification reaction, and preferably in an amplification reaction in a single vessel.

In another aspect, the primers used for amplification of the selected nucleic acid regions are designed to provide a random index between the selected region complementary to the biological molecule and a universal amplification region. In such an aspect, a sufficient number of identification indices are present to uniquely identify each selected nucleic acid region in the sample. Each nucleic acid region to be analyzed is associated with a unique identification index, so that the identification index is uniquely associated with the selected nucleic acid region. Quantification of the identification index in a sample provides quantification data for the associated selected nucleic acid region and the chromosome corresponding to the selected nucleic acid region. The identification locus may also be used to detect any amplification bias that occurs downstream of the initial isolation of the selected nucleic acid regions from a sample.

In certain aspects, only the locus index and/or the identification index (if present) are detected and used to quantify the selected nucleic acid regions in a sample. In another aspect, a count of the number of times each locus index occurs with a unique identification index is done to determine the relative frequency of a selected nucleic acid region in a sample.

In addition to locus-specific indices and identification indices, additional indices can be introduced to biological molecules during an amplification or sequencing reaction to assist in the multiplexing of samples. In addition, indices which identify sequencing error, which allow for highly multiplexed amplification techniques or which allow for hybridization or ligation or attachment to another surface can be added to the primers. The order and placement of these indices, as well as the length of these indices, can vary.

Preferably, the index region corresponds to the selected nucleic acid region, so that identification of the index region can be used as a surrogate for detection of the actual sequence of the selected nucleic acid region. The index region may optionally comprise a sample index to identify the oligo set as being from a particular sample in a multiplexed assay system.

### Amplification and Sequence Determination

In a preferred aspect, the multiplex amplification products are quantified by analysis of the amplification products. In a preferred aspect, a representational sample of individual molecules from the amplification processes is isolated from the other molecules for further analysis. To obtain a representational sample of individual molecules, the average number of molecules per locus must exceed the sampling noise created by the multiplexed reaction. In one aspect, the average number per locus is greater than 100. In another aspect, the average number per locus is greater than 500. In another aspect the average number per locus is greater than 1000.

Individual molecules from the amplification product are preferably isolated physically from the other molecules in a manner that allows the different amplification products to be distinguished from one another in analysis. In a preferred aspect, this isolation occurs on a solid substrate. The isolated molecule may be associated with a particular identifiable or physical address either prior to analysis, or the address may become known for the particular amplification products based on the outcome of the analysis. The substrate may be a planar surface or three-dimensional surface such as a bead.

Once isolated, the individual amplification product may be further amplified to make multiple identical copies of that molecule at the same known or identifiable location. The amplification may occur before or after that location becomes an identifiable or physical address. The amplification product and or its copies (which may be identical or complementary to the amplification product) are then analyzed based on the sequence of the amplification product or its copies to identify the particular locus and/or allele it represents.

In a preferred aspect, the entire length of the amplification product or a portion of the amplification product may be analyzed using sequence determination. The number of bases that need to be determined must be sufficient to uniquely identify the amplification product as belonging to a specific locus and/or allele. In one preferred aspect, the amplification product is analyzed through sequence determination of the selected amplification product.

Numerous methods of sequence determination are compatible with the assay systems of the inventions. Exemplary methods for sequence determination include, but are not limited to, including, but not limited to, hybridization-based methods, such as disclosed in Drmanac, U.S. Pat. Nos. 6,864,052; 6,309,824; and 6,401,267; and Drmanac et al, U.S. patent publication 2005/0191656, sequencing by synthesis methods, e.g., Nyren et al, U.S. Pat. No. 7,648,824, 7,459,311 and 6,210,891; Balasubramanian, U.S. Pat. Nos. 7,232,656 and 6,833,246; Quake, U.S. Pat. No. 6,911,345; Li et al, Proc. Natl. Acad. Sci., 100: 414-419 (2003); pyrophosphate sequencing as described in Ronaghi et al., U.S. Pat. Nos. 7,648,824, 7,459,311, 6,828,100, and 6,210,891; and ligation-based sequencing determination methods, e.g., Drmanac et al., U.S. Pat. Appln No. 20100105052, and Church et al, U.S. Pat. Appln Nos. 20070207482 and 20090018024.

Sequence information may be determined using methods that determine many (typically thousands to billions) of nucleic acid sequences in an intrinsically parallel manner, where many sequences are read out preferably in parallel using a high throughput serial process. Such methods include but are not limited to pyrosequencing (for example, as commercialized by 454 Life Sciences, Inc., Branford, CT); sequencing by ligation (for example, as commercialized in the SOLiD^{™} technology, Life Technology, Inc., Carlsbad, CA); sequencing by synthesis using modified nucleotides (such as commercialized in TruSeqTM and HiSeq^{™} technology by Illumina, Inc., San Diego, CA, HeliScope^{™} by Helicos Biosciences Corporation, Cambridge, MA, and PacBio RS by Pacific Biosciences of California, Inc., Menlo Park, CA), sequencing by ion detection technologies (Ion Torrent, Inc., South San Francisco, CA); sequencing of DNA nanoballs (Complete Genomics, Inc., Mountain View, CA); nanopore-based sequencing technologies (for example, as developed by Oxford Nanopore Technologies, LTD, Oxford, UK), and like highly parallelized sequencing methods.

Alternatively, in another aspect, the entire length of the amplification product or a portion of the amplification product may be analyzed using hybridization techniques. Methods for conducting polynucleotide hybridization assays for detection of have been well developed in the art. Hybridization assay procedures and conditions will vary depending on the application and are selected in accordance with the general binding methods known including those referred to in: Maniatis et al. Molecular Cloning: A Laboratory Manual (2nd Ed. Cold Spring Harbor, N.Y., 1989); Berger and Kimmel Methods in Enzymology, Vol. 152, Guide to Molecular Cloning Techniques (Academic Press, Inc., San Diego, Calif., 1987); Young and Davis, P.N.A.S, 80: 1194 (1983). Methods and apparatus for carrying out repeated and controlled hybridization reactions have been described in U.S. Pat. Nos. 5,871,928, 5,874,219, 6,045,996 and 6,386,749, 6,391,623 each of which are incorporated herein by reference.

The present invention also contemplates signal detection of hybridization between ligands in certain preferred aspects. See U.S. Pat. Nos. 5,143,854, 5,578,832; 5,631,734; 5,834,758; 5,936,324; 5,981,956; 6,025,601; 6,141,096; 6,185,030; 6,201,639; 6,218,803; and 6,225,625, in U.S. Patent application 60/364,731 and in PCT Application PCT/US99/06097 (published as WO99/47964),

Methods and apparatus for signal detection and processing of intensity data are disclosed in, for example, U.S. Pat. Nos. 5,143,854, 5,547,839, 5,578,832, 5,631,734, 5,800,992, 5,834,758; 5,856,092, 5,902,723, 5,936,324, 5,981,956, 6,025,601, 6,090,555, 6,141,096, 6,185,030, 6,201,639; 6,218,803; and 6,225,625, in U.S. Patent application 60/364,731 and in PCT Application PCT/US99/06097 (published as WO99/47964),

### Use of the Processes in Detection of Fetal Abnormalities

Chromosome abnormalities, including aneuploidies, duplications, translocations and the like, account for a wide number of pathologies, including syndromes caused by chromosomal aneuploidy (e.g., Down syndrome) and those caused by subchromosomal abnormalities (e.g., DiGeorge syndrome). Methods for determining genetic anomalies have become standard techniques for identifying specific syndromes, diseases and disorders. Detection of gross chromosomal abnormalities, such as trisomies, monosomies, translocations and large insertions or deletions have become standard practice in high-risk populations to determine the presence or absence of certain disorders. For example, chromosomal abnormalities such as trisomies 13, 18, and 21, the Robertsonian translocation associated with certain forms of Down syndrome, and larger deletions such as those found on chromosome 22 in DiGeorge syndrome all impact significantly on fetal health.

The processes of the invention are used in the normalization of multiplexed data sets for the identification of the presence or absence of one or more chromosomal abnormalities in a fetus using a maternal sample. Preferably, the maternal sample is blood, serum or plasma. In some aspects, the maternal sample comprises maternal and fetal cells. In other aspects, the maternal sample comprises maternal and fetal cell-free nucleic acids, such as RNA or DNA. Preferably, the maternal sample comprises cell free DNA.

### EXAMPLES

The following examples are put forth so as to provide those of ordinary skill in the art with a complete disclosure and description of how to make and use the present invention, and are not intended to limit the scope of what the inventors regard as their invention, nor are they intended to represent or imply that the experiments below are all of or the only experiments performed.

Efforts have been made to ensure accuracy with respect to numbers used (*e.g*., amounts, temperature, etc.) but some experimental errors and deviations should be accounted for. Unless indicated otherwise, parts are parts by weight, molecular weight is weight average molecular weight, temperature is in degrees centigrade, and pressure is at or near atmospheric.

### Example 1: General Aspects of the Processes of the Invention

To assess chromosome proportion, assays were performed against 576 non-polymorphic loci on each of chromosome 18 and chromosome 21, where each assay consisted of three locus specific oligonucleotides: a left oligo with a 5' universal amplification tail, a 5' phosphorylated middle oligo, and a 5' phosphorylated right oligo with a 3' universal amplification tail. To assess fetal fraction, we designed assays against a set of 192 SNP-containing loci on chr1-12, where two middle oligos, differing by one base, were used to query each SNP. SNPs were optimized for minor allele frequency in the HapMap 3 dataset. Oligonucleotides were synthesized by IDT and pooled together to create a single multiplexed DANSR assay pool.

Products from 96 independent samples were pooled and used as template for cluster amplification on a single lane of a TruSeq v2 SR flow slide (Illumina, San Diego, CA). The slide was processed on an Illumina HiSeq 2000 to generate an average of 1.18M raw sequencing reads/sample. An average of 1.15M (97%) reads had fewer than 3 mismatches with expected assay structures, resulting in an average of 854 reads/locus/sample.

### Example 2: Sample Effect Removal

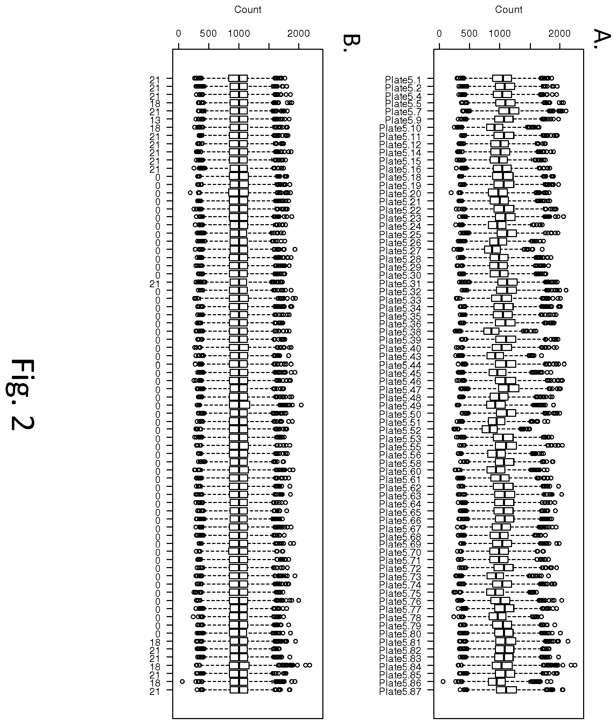

In a first example, the processes of the invention were utilized to remove variations in sequence counts between multiple samples in a multiplexed sequence data set. The raw per-sample sequence counts were determined as per Example 1. FIG. 2 is a plot of such determined sequences. Each box plot demonstrates the raw, unadjusted sequence counts for all chromosomes within a sample, with each smaller box representing a set of all loci for a given sample. As illustrated, certain samples generated more or less median sequence counts than other samples. In the bottom panel, the same samples are plotted after median-centering normalization by scaling each sample's median count to a reference count of 1000. Noticeably, the systematic biases pertaining to certain samples were removed.

### Example 3: Locus Effect Removal

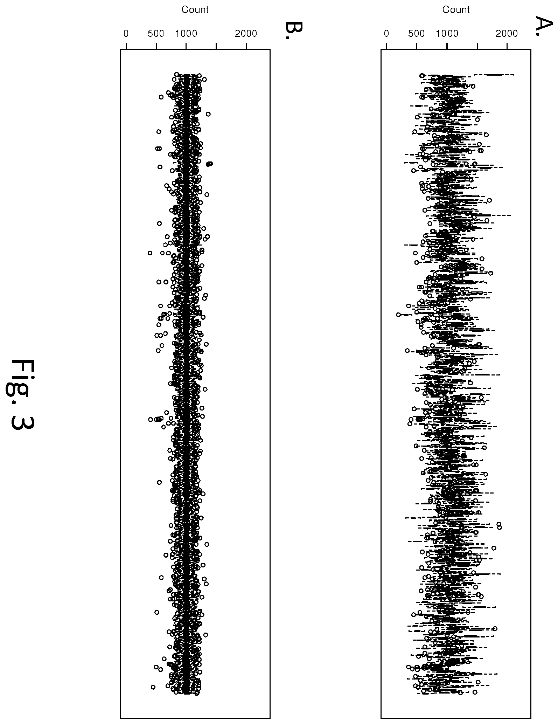

In a next example, sequences from a multiplexed sequence data set with counts representing a single locus were normalized using the processes of the invention. The processes of the invention were utilized to remove variations in sequence counts between the same locus from various samples. Raw per-locus sequence counts for chromosome 21 determined as per Example 2 are plotted as box-plots in FIG.3A. Each box is a plot of all samples for a given locus. Each box is a plot of all samples for a given locus. FIG.3B illustrates the same loci in FIG.A from chromosome 21 after normalization was performed using the Median-Polish algorithm [Tukey, JW. Exploratory Data Analysis. Reading Massachusetts: Addison-Wesley. 1977] with other sequences within the multiplexed data set. Noticeably, the systematic biases pertaining to certain loci were removed.

### Example 4: Examples of chromosomal level normalization.

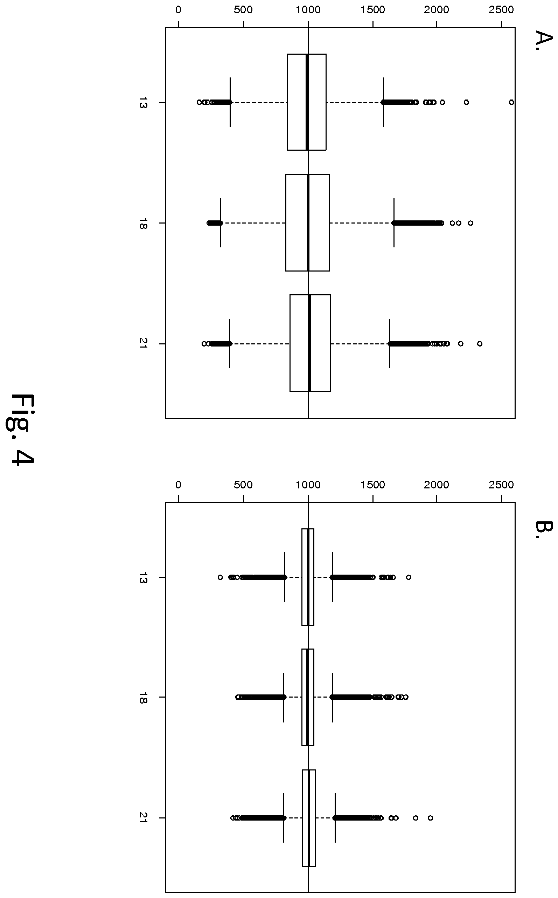

In another example, bias may be introduced into multiplexed data sets on a per chromosome basis. The processes of the invention using internal normalization can be used to reduce such bias as shown in FIGs.4A and 4B. FIG.4A shows the level of sequence counts after initial median-centering normalization. FIG.4B shows the level of per-chromosome sequence counts across the multiplexed dataset after normalization. The premise for this normalization is that the median sequence counts across the chromosomes should be the same. However, sometimes one chromosome tends to generate more sequence counts than another chromosome not because of underlying biology but because of other technical reasons. This effect is removed as part of the RMA normalization using other sequences within the multiplexed data set.

### Example 5: Normalization based on chromosome position.

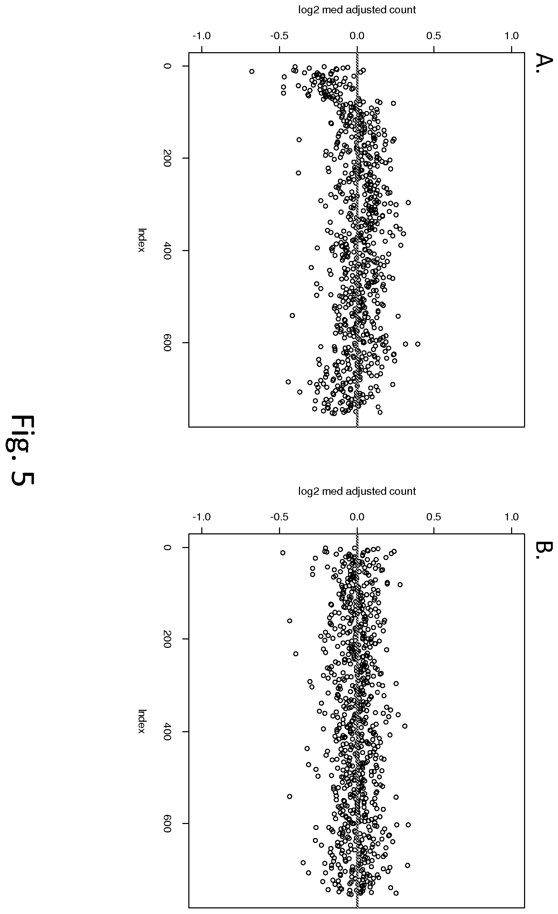

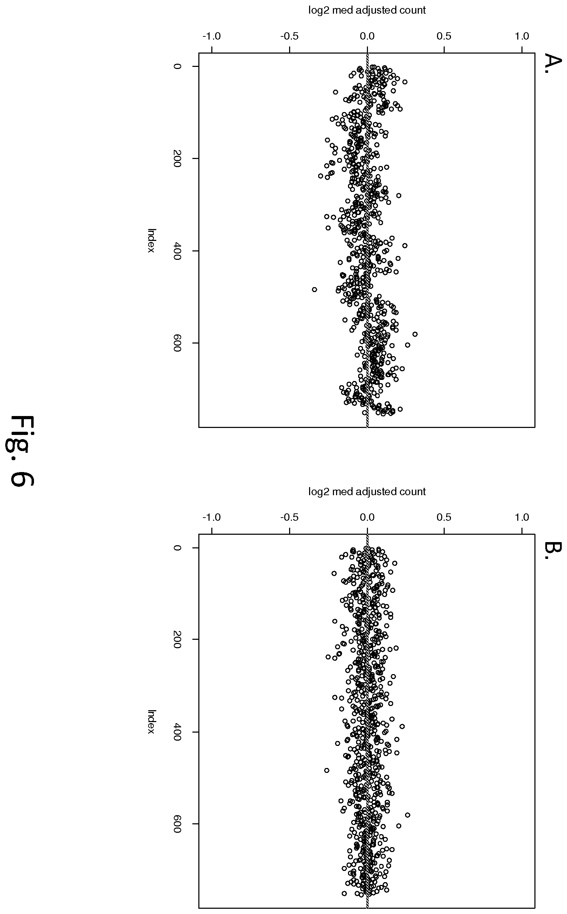

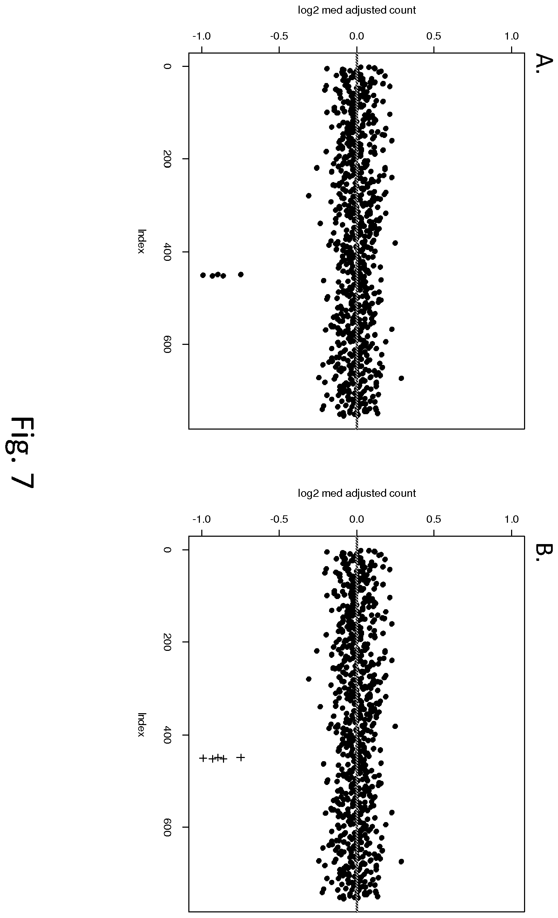

In some aspects, certain regions of chromosome appeared to have lower sequence counts, which does not have any relation to the actual ploidy state of the fetus. This variability was normalized using the internal sequences to the multiplexed dataset for LOcally WEighted Scatterplot Smoothing (LOWESS) [Cleveland, W. S. (1979) Robust locally weighted regression and smoothing scatterplots. J. Amer. Statist. Assoc. 74, 829-836.; Cleveland, W. S. (1981) LOWESS: A program for smoothing scatterplots by robust locally weighted regression. The American Statistician, 35, 54]. LOWESS is a modern nonlinear regression technique that smooth out systematic and unwanted structures in the data. Briefly, a small subset of the detected loci within a multiplexed set were used to construct a local linear regression model. The modeled values were then removed from the sequence counts, thereby smoothing out the structural biases. FIG. 5A illustrates the data from a multiplexed sequence data set corresponding to sequences from chromosome 18 before LOWESS smoothing, and FIG.5B illustrates the same data after LOWESS smoothing. FIG. 6A illustrates the data from a multiplexed sequence data set corresponding to sequences from chromosome 21 before LOWESS smoothing, and FIG.6B illustrates the same data after LOWESS smoothing. In FIGs.5A, 5B, 6A, 6B, counts were divided by the median count in the chromosomes and the ratio transformed by the logarithm base 2 for better visualization.

## Claims

1. A computer implemented process for the identification of the presence or absence of one or more chromosomal abnormalities in a fetus using a maternal sample comprising both fetal and maternal nucleic acids, the process comprising:
i) providinga data set comprising frequency sequence data for a set of at least 16 loci from a single chromosome from two or more maternal samples each comprising both fetal and maternal nucleic acids, wherein each sample is identified by a sample index, and wherein the sequences of the dataset are determined in a single sequencing reaction;
ii) normalizing the frequency of a sequence of individual loci within said dataset;
wherein locus or loci refers to a nucleic acid region of known location in a genome, and wherein the normalizing comprises:
subjecting the detected frequencies of a sequence from an individual locus across all samples to a mathematical transformation based on the frequencies of sequences of the at least 15 other loci across all samples within the data set, to reduce experimentally introduced variation,
wherein the mathematical transformation comprises using a regression technique to normalize the frequency of the set of loci; and
iii) identifying differences in sequence frequencies of a normalized locus from the fetal chromosome relative to sequence frequencies of loci of one or more other chromosomes in one or more of said maternal samples, thereby identifying the presence or absence of one or more chromosomal abnormalities in a fetus.

2. The process of claim 1, wherein the nucleic acids are DNA.

3. The process of claim 1, wherein the regression technique is multiple linear regression or median polish.

4. The process of claim 1, wherein the sequences of the individual loci within the data set have been subjected to an amplification reaction prior to sequence determination.

5. The process of claim 4, wherein the amplification reaction uses primers that comprise both an index region and a sequence region that specifically hybridizes to the individual locus.

6. The process of claim 1, wherein sequence data on at least 64 loci are used in the process.

7. The process of claim 6, wherein sequence data on at least 384 loci are used in the process.

8. The process of claim 7, wherein sequence data on at least 768 loci are used in the process.

## Patentansprüche

1. Computerimplementiertes Verfahren zur Identifizierung des Vorliegens oder Nicht-Vorliegens von einer oder mehreren chromosomalen Abnormitäten bei einem Fötus unter Verwendung einer mütterlichen Probe, die sowohl fötale als auch mütterliche Nucleinsäuren umfasst, wobei das Verfahren Folgendes umfasst:
i) das Bereitstellen eines Datensatzes, der Häufigkeitssequenzdaten für einen Satz von zumindest 16 Loci von einem einzigen Chromosom aus zwei oder mehr mütterlichen Proben umfasst, wobei jede sowohl fötale als auch mütterliche Nucleinsäuren umfasst, wobei jede Probe durch einen Probenindex identifiziert ist und wobei die Sequenzen des Datensatzes in einer einzigen Sequenzierungsreaktion bestimmt wird;
ii) das Normalisieren der Häufigkeit einer Sequenz einzelner Loci innerhalb des Datensatzes;
wobei Locus oder Loci sich auf eine Nucleinsäureregion bezieht, deren Position in einem Genom bekannt ist, und wobei das Normalisieren Folgendes umfasst:
das Unterziehen der detektierten Häufigkeiten einer Sequenz von einem einzelnen Locus in allen Proben einer mathematischen Transformation auf Grundlage der Häufigkeiten der Sequenzen der zumindest 15 anderen Loci in allen Proben innerhalb des Datensatzes, um experimentell verursachte Variation zu reduzieren,
wobei die mathematische Transformation das Anwenden eines Regressionsverfahrens zum Normalisieren der Häufigkeit des Satzes von Loci umfasst; und
iii) das Identifizieren von Unterschieden der Sequenzhäufigkeiten eines normalisierten Locus aus dem fötalen Chromosom im Vergleich zu den Sequenzhäufigkeiten von Loci von einem oder mehreren anderen Chromosomen in einer oder mehreren der mütterlichen Proben, wodurch das Vorliegen oder Nicht-Vorliegen von einer oder mehreren chromosomalen Abnormitäten bei einem Fötus identifiziert wird.

2. Verfahren nach Anspruch 1, wobei die Nucleinsäuren DNA sind.

3. Verfahren nach Anspruch 1, wobei das Regressionsverfahren multiple lineare Regression oder Medianpolieren (Median Polish) ist.

4. Verfahren nach Anspruch 1, wobei die Sequenzen der einzelnen Loci in dem Datensatz vor der Sequenzbestimmung einer Amplifikationsreaktion unterzogen werden.

5. Verfahren nach Anspruch 4, wobei in der Amplifikationsreaktion Primer verwendet werden, die sowohl eine Indexregion als auch eine Sequenzregion umfassen, die spezifisch an den einzelnen Locus hybridisiert.

6. Verfahren nach Anspruch 1, wobei Sequenzdaten für zumindest 64 Loci in dem Verfahren verwendet werden.

7. Verfahren nach Anspruch 6, wobei Sequenzdaten für zumindest 384 Loci in dem Verfahren verwendet werden.

8. Verfahren nach Anspruch 7, wobei Sequenzdaten für zumindest 768 Loci in dem Verfahren verwendet werden.

## Revendications

1. Procédé mis en œuvre par ordinateur pour l'identification de la présence ou de l'absence d'une ou plusieurs anomalies chromosomiques chez un fœtus en utilisant un échantillon maternel comprenant à la fois des acides nucléiques fœtaux et maternels, le procédé comprenant les étapes consistant à :
i) fournir un ensemble de données comprenant des données de séquence de fréquence pour un ensemble d'au moins 16 loci à partir d'un seul chromosome provenant de deux échantillons maternels ou plus, chacun comprenant à la fois des acides nucléiques fœtaux et maternels, dans lequel chaque échantillon est identifié par un indice d'échantillon, et dans lequel les séquences de l'ensemble de données sont déterminées en une réaction de séquençage unique ;
ii) normaliser la fréquence d'une séquence de loci individuels dans ledit ensemble de données ;
dans lequel le locus ou les loci font référence à une région d'acide nucléique d'un emplacement connu dans un génome, et dans lequel la normalisation comprend l'étape consistant à :
soumettre les fréquences détectées d'une séquence à partir d'un locus individuel à travers tous les échantillons à une transformation mathématique sur la base des fréquences de séquences des au moins 15 autres loci à travers tous les échantillons dans l'ensemble de données, pour réduire une variation introduite de manière expérimentale,
dans lequel la transformation mathématique comprend l'utilisation d'une technique de régression pour normaliser la fréquence de l'ensemble de loci ; et
iii) identifier des différences dans des fréquences de séquence d'un locus normalisé à partir du chromosome fœtal par rapport à des fréquences de séquence de loci d'un ou plusieurs autres chromosomes dans un ou plusieurs desdits échantillons maternels, en identifiant ainsi la présence ou l'absence des une ou plusieurs anomalies chromosomiques chez un fœtus.

2. Procédé selon la revendication 1, dans lequel les acides nucléiques sont de l'ADN.

3. Procédé selon la revendication 1, dans lequel la technique de régression est une régression linéaire multiple ou un polissage médian.

4. Procédé selon la revendication 1, dans lequel les séquences des loci individuels dans l'ensemble de données multiplexées ont été soumises à une réaction d'amplification avant une détermination de séquence.

5. Procédé selon la revendication 4, dans lequel la réaction d'amplification utilise des amorces qui comprennent à la fois une région d'index et une région de séquence qui s'hybride spécifiquement au locus individuel.

6. Procédé selon la revendication 1, dans lequel des données de séquence sur au moins 64 loci sont utilisées dans le procédé.

7. Procédé selon la revendication 6, dans lequel des données de séquence sur au moins 384 loci sont utilisées dans le procédé.

8. Procédé selon la revendication 7, dans lequel des données de séquence sur au moins 768 loci sont utilisées dans le procédé.
